(19) Europäisches Patentamt

European Patent Office

Office européen des brevets

(11) **EP 4 696 750 A1**

(12) **EUROPÄISCHE PATENTANMELDUNG**

(43) Veröffentlichungstag:
**18.02.2026   Patentblatt 2026/08**

(21) Anmeldenummer: **25191356.2**

(22) Anmeldetag: **23.07.2025**

(51) Internationale Patentklassifikation (IPC):
$C09C\ 1/00\ ^{(2006.01)}$        $A61K\ 8/19\ ^{(2006.01)}$
$C04B\ 35/00\ ^{(2006.01)}$      $C08K\ 3/01\ ^{(2018.01)}$
$C08K\ 3/013\ ^{(2018.01)}$      $C09D\ 5/36\ ^{(2006.01)}$

(84) Benannte Vertragsstaaten:
**AL AT BE BG CH CY CZ DE DK EE ES FI FR GB
GR HR HU IE IS IT LI LT LU LV MC ME MK MT NL
NO PL PT RO RS SE SI SK SM TR**
Benannte Erstreckungsstaaten:
**BA**
Benannte Validierungsstaaten:
**GE KH LA MA MD TN**

(30) Priorität:   **16.08.2024   EP 24195014**

(71) Anmelder: **SUSONITY Commercial GmbH
64579 Gernsheim (DE)**

(72) Erfinder:
• **Handrosch, Carsten
  Darmstadt (DE)**
• **Knapp, Martin
  Darmstadt (DE)**
• **Plueg, Carsten
  Darmstadt (DE)**
• **Schoen, Sabine
  Darmstadt (DE)**
• **Dobelmann-Mara, Lars
  Darmstadt (DE)**

(74) Vertreter: **Merck Patent Association
Merck Patent GmbH
64271 Darmstadt (DE)**

(54) **INTERFERENZPIGMENTE**

(57)   Die vorliegende Erfindung betrifft Interferenzpigmente basierend auf $SiO_2$-Plättchen, die mit Kristalliten des alpha-$Fe_2O_3$ beschichtet sind, sowie deren Verwendung, insbesondere in Farben, Lacken, Industrie- und Automobillacken, keramischen Materialien, Kunststoffen und kosmetischen Formulierungen.

EP 4 696 750 A1

# EP 4 696 750 A1

**Beschreibung**

Die vorliegende Erfindung betrifft Interferenzpigmente basierend auf $SiO_2$-Plättchen, die mit alpha-$Fe_2O_3$-Kristalliten beschichtet sind, und die Verwendung der Pigmente, insbesondere in Farben, Lacken, Druckfarben, Kunststoffen und kosmetischen Formulierungen.

Aus der WO 93/08237 sind Interferenzpigmente auf der Basis von transparenten Siliziumdioxid-Plättchen bekannt. Die dort beschriebenen Pigmente basieren auf $SiO_2$-Plättchen, die mit ein oder mehreren Metalloxidschichten beschichtet sind. Anstelle von Metalloxiden können die $SiO_2$-Plättchen auch mit anderen Materialien, wie beispielsweise Metallen, Sulfiden oder Nitriden, umhüllt sein. Farbstarke rote Interferenzpigmente basierend auf $SiO_2$-Plättchen, die mit Eisenoxid beschichtet sind, sind beispielsweise Gegenstand der WO 2007/057111 A2 und EP 1 681 318 A2.

Die aus dem Stand der Technik bekannten roten Interferenzpigmente zeigen einen starken orange-roten Farbeffekt im steilen Betrachtungswinkel, wobei es sich um eine Kombination aus Interferenz- und Absorptionsfarbe handelt, und ein sehr hohes Deckvermögen.

Aufgabe der vorliegenden Erfindung ist es ein hoch deckendes körperfarbenes Interferenzpigment mit einer Kombination aus brilliant bläulich-roter Absorptionsfarbe und hoch-chromatischer bläulich-roter Interferenzfarbe bereitzustellen, bei dem ein geringer Farbflop auftritt und demnach auch im flachem Betrachtungswinkel die bläulich-rote Absorptionsfarbe sichtbar bleibt, so dass die bei flachem Betrachtungswinkel naturgemäß abnehmende Intensität der Interferenzfarbe durch die unter allen Betrachtungswinkeln brilliante Absorptionsfarbe ausgeglichen wird. Als Folge davon ist kein oranger oder gar dunkel-brauner down-flop zu beobachten. Insbesondere soll ein Interferenzpigment zur Verfügung gestellt werden, welches unter allen Betrachtungswinkeln einen brillianten Rot-Farbton aufweist. Weiterhin sollte das Pigment hohes Deckvermögen bei gleichzeitig geringer Textur aufweisen und für vegane Anwendungen einsetzbar ein.

Überraschenderweise wurden gefunden, dass Interferenzpigmente basierend auf $SiO_2$-Plättchen und beschichtet mit alpha-$Fe_2O_3$-Kristalliten definierter Größe keine der oben genannten Nachteile aufweisen und unter allen Betrachtungswinkel einen reinen und brillianten bläulich-roten Farbton zeigen. Die Pigmente zeichnen sich durch ihr relativ hohes Deckvermögen und die hohe Farbsättigung aus, sind temperaturstabil und lassen sich universell in den verschiedensten Anwendungen einsetzen.

Die erfindungsgemäßen Interferenzpigmente zeigen auf allen Untergründen (weiß/schwarz) eine deckende, farbreine, bläulich-rote Absorptionsfarbe und besitzen darüber hinaus nur eine geringe Winkelabhängigkeit der überlagerten Interferenzfarbe. Somit ergibt sich als erfindungsgemäßer Gesamtfarbeffekt (Absorptivfarbe + Interferenzfarbe) ein unter allen Betrachtungswinkeln (steil/flach) farbreiner, bläulich-roter Farbton. Aus anwendungstechnischer Sicht sind die erfindungsgemäßen Interferenzpigmente mit der Kombination aus:

- chromatischer, blauroter Körperfarbe
- intensiver bläulich-roter Interferenzfarbe
- geringer Winkelabhängigkeit des Farbeindruckes / geringer Farbflop
- hohes Deckvermögen

insbesondere geeignet für die Farbgebung von Lackformulierungen, Farben, und Kunststoffen.

Ferner bietet sich aufgrund des sehr guten Hautgefühls die Anwendung in kosmetischen Formulierungen an. Ganz besonders von Vorteil ist in diesem Anwendungsbereich die Möglichkeit in Abmischungen mit Weißpigmenten um vegane Kosmetika herzustellen, da durch diese Pigmentmischungen in pinkfarbenen Formulierungen das klassische Karminrot ersetzt werden kann.

Gegenstand der vorliegenden Erfindung sind Interferenzpigmente basierend auf $SiO_2$-Plättchen, dadurch gekennzeichnet, dass sie mit Kristalliten aus alpha-Hämatit (alpha-$Fe_2O_3$) belegt sind.

Aufgrund der vorteilhaften Eigenschaften eignen sich die erfindungsgemäßen Interferenzpigmente universell für eine große Anzahl unterschiedlichster Anwendungen. Gegenstand der vorliegenden Erfindung ist demgemäß auch die Verwendung dieser Pigmente in Farben, Lacken, Industrie- und Automobillacken, Druckfarben, Sicherheitsdruckfarben, Papier, Kunststoffen, Folien, kosmetischen Formulierungen, Knopfpasten, Pigmentgemischen, Trockenpräparaten oder Pigmentpräparationen, keramischen Materialien, keramischen Farben, Glasuren, Engoben, Emaillen und Gläsern, als Absorber zur Lasermarkierung von Kunststoffen, zur Lebensmitteleinfärbung, zur Veredlung von Lebensmitteln, in Lebensmittel- und Arzneimittelüberzügen, für Sicherheitsmerkmale in Dokumenten und Ausweisen, zur Saatguteinfärbung, für RADAR-Anwendungen, zur Farbgebung von Solarzellen.

Die erfindungsgemäßen Pigmente basieren auf synthetischen $SiO_2$-Plättchen als Substrat, die in der Regel über eine einheitliche Schichtdicke verfügen und vorzugsweise gemäß der internationalen Anmeldung WO 93/08237 auf einem endlosen Band durch Verfestigung und Hydrolyse einer Wasserglaslösung hergestellt werden. Unter einheitlicher Schichtdicke wird dabei eine Schichtdickentoleranz von 3 bis 10 %, bevorzugt von 3 bis 5 %, der Gesamttrockenschichtdicke der Partikel verstanden. Die plättchenförmigen Siliziumdioxidpartikel liegen im allgemeinen in amorpher Form vor. Synthetische Plättchen dieser Art haben gegenüber natürlichen Materialien, wie z.B. Glimmer, den Vorteil, dass die Schichtdicke im Hinblick auf die gewünschten Effekte eingestellt werden kann und die Schichtdickentoleranz begrenzt ist.

Geeignete $SiO_2$-Plättchen für die erfindungsgemäßen Interferenzpigmente haben vorzugsweise einen Durchmesser von 1 bis 250 $\mu$m, insbesondere von 1 - 65 $\mu$m. Die Dicke der $SiO_2$-Plättchen Dicke beträgt vorzugsweise 250 - 500 nm, insbesondere 330 bis 400 nm und die mittlere Schichtdicke der $\alpha$-$Fe_2O_3$-Schicht im Pigment beträgt 85 +/-35 nm.

Wesentlich für die erfindungsgemäßen Interferenzpigmente sind die Kristallite auf der Oberfläche der $SiO_2$-Plättchen. Die Kristallite liegen ungleichmäßig verteilt auf der Oberfläche der $SiO_2$-Plättchen vor und bestehen aus $\alpha$-$Fe_2O_3$. Die mittlere Größe der Kristallite beträgt vorzugsweise $\geq$ 45 nm, insbesondere $\geq$ 50 nm.

Die Keimbildung und Kristallitgröße kann u.a. durch die Reaktionstemperatur und die Rührgeschwindigkeit sowie die Dauer der Zudosierung und durch die Glühtemperatur und Verweilzeit im Glühofen beeinflusst bzw. eingestellt werden.

Neben den Kristalliten und deren Größe ist die Gesamtdicke des Interferenzpigments wesentlich für die Eigenschaften des Pigments. Die Dicke der $SiO_2$-Plättchen und der Kristallit-Schicht ist vorzugsweise so gewählt, dass die Gesamtdicke der Interferenzpigmente im ungewichteten arithmetischen Mittel nicht größer als 494 nm (+/- 109 nm) ist.

Die Belegung der $SiO_2$-Plättchen auf der Oberfläche mit den Kristalliten des $\alpha$-$Fe_2O_3$ kann nasschemisch und/oder durch CVD- oder PVD-Verfahren erfolgen. Vorzugsweise werden die erfindungsgemäßen Interferenzpigmente nasschemisch hergestellt, bei denen die bekannten, zur Herstellung von Perlglanzpigmenten entwickelten nasschemischen Beschichtungstechnologien angewendet werden können.

Zur Beschichtung werden die $SiO_2$-Plättchen in Wasser suspendiert und durch langsame und kontrollierte Zugabe und Fällung entsprechender anorganischer Eisenverbindungen beschichtet, wobei durch gleichzeitige Zugabe von Säure oder Base der für die Fällung von $\alpha$-$Fe_2O_3$ und die Keimbildung notwendige pH-Wert eingestellt und konstant gehalten wird, und anschließend das mit den Kristalliten belegte Substrat aus der wässrigen Suspension abgetrennt, getrocknet und kalziniert wird.

Die Kalzinierungstemperatur beträgt 700 bis 1000 °C, vorzugsweise 800 bis 950 °C, um zu gewährleisten, dass die Kristallite kein FeOOH enthalten, sondern ausschließlich aus $\alpha$-$Fe_2O_3$ bestehen. Durch die hohe Kalzinierungstemperatur findet auch eine höhere Verdichtung der Kristallite statt, was wiederum zu einem höheren Brechungsindex führt. Letzterer ist u. a. verantwortlich für die hohe Luminanz der erfindungsgemäßen Interferenzpigmente. Die Dauer des Glühprozesses beträgt in der Regel 30 bis 60 Minuten. Eine längere Verweildauer im Glühofen ist vorteilhaft für die gewünschte Verdichtung der Kristallite.

Für die Herstellung der erfindungsgemäßen Interferenzpigmente sind auch CVD- oder PVD-Verfahren zur Beschichtung der $SiO_2$-Plättchen mit Kristalliten des $\alpha$-$Fe_2O_3$ geeignet.

Zur Erhöhung der Licht-, Wasser- und Wetterstabilität empfiehlt es sich häufig, in Abhängigkeit vom Einsatzgebiet, das erfindungsgemäße Interferenzpigment einer anorganischen oder organischen Nachbeschichtung oder Nachbehandlung zu unterziehen. Als Nachbeschichtungen bzw. Nachbehandlungen kommen beispielsweise die in den EP 0 632 109, US 5,759,255, DE 43 17 019, DE 39 29 423, DE 32 35 017, EP 0 492 223, EP 0 342 533, EP 0 268 918, EP 0 141 174, EP 0 764 191, WO 98/13426 oder EP 0 465 805, deren Offenbarung hiermit unter Bezugnahme mit eingeschlossen ist, beschriebenen Verfahren in Frage. Durch diese Nachbeschichtung wird die chemische und photochemische Stabilität weiter erhöht oder die Handhabung des Interferenzpigments, insbesondere die Einarbeitung in unterschiedliche Medien, erleichtert. Zur Verbesserung der Benetzbarkeit, Dispergierbarkeit und/oder Verträglichkeit mit den Anwendermedien können funktionelle Beschichtungen aus $SiO_2$, $SnO_2$, $Al_2O_3$ oder $ZrO_2$ oder deren Gemische auf die Pigmentoberfläche aufgebracht werden. Weiterhin sind organische Nachbeschichtungen möglich, z.B. mit Silanen, wie beispielsweise beschrieben in der EP 0090259, EP 0 634 459, WO 99/57204, WO 96/32446, WO 99/57204, U.S. 5,759,255, U.S. 5,571,851, WO 01/92425 oder in J.J. Ponjeé, Philips Technical Review, Vol. 44, No. 3, 81 ff. und P.H. Harding J.C. Berg, J. Adhesion Sci. Technol. Vol. 11 No. 4, S. 471-493. Interferenzpigmente enthaltend eine organische Beschichtung, z.B. aus Organosilanen oder Organotitanaten bzw. Organozirkonaten zeigen neben den bereits genannten optischen Eigenschaften zusätzlich eine erhöhte Stabilität gegenüber Witterungseinflüssen, wie z.B. Feuchtigkeit und Licht, was vor allem für Industrielacke und im Automobilbereich von besonderem Interesse ist. Die Stabilisierung kann durch anorganische Komponenten der zusätzlichen Beschichtung verbessert werden. Insgesamt sind die jeweiligen Anteile für die zusätzliche stabilisierende Beschichtung so auszuwählen, dass die optischen Eigenschaften der erfindungsgemäßen Interferenzpigmente nicht wesentlich beeinflusst werden. Die hierbei aufgebrachten Stoffe umfassen lediglich einen Gewichtsanteil von 0,1 bis 5 Gew. %, vorzugsweise 0,5 bis 3 Gew. %, des gesamten Pigmentes.

Die Nachbeschichtung der erfindungsgemäßen Interferenzpigmente kann direkt in einem Eintopfverfahren an die $\alpha$-$Fe_2O_3$-Kristallit-Beschichtung der $SiO_2$-Plättchen erfolgen. Es ist aber auch möglich das Interferenzpigment zunächst zu isolieren, zu trocken und zu kalzinieren und anschließend die Nachbeschichtung aufzubringen.

Unter Beschichtung(en) sind in dieser Patentanmeldung die vollständige Belegung/Umhüllung der plättchenförmigen Substrate zu verstehen.

Das Deckvermögen der erfindungsgemäßen Interferenzpigmente kann weiterhin erhöht werden, wenn die Pigmente in Kombination mit organischen und anorganischen Füllstoffen und/oder mit plättchenförmigen, nadelförmigen, sphärischen oder kristallinen Farbmitteln gemischt werden. Durch die Zumischung eines oder mehrerer Farbmittel zu den erfindungsgemäßen Interferenzpigmenten können Farbeffekte verstärkt und neuartige Farbeffekte erzielt werden. Gegenstand der vorliegenden Erfindung sind daher ebenfalls Pigmentgemische.

Die erfindungsgemäßen Interferenzpigmente zeigen durch die messbar größere mittlere Kristallitgröße eine erhöhte Temperatur- und Hitzestabilität auf im Vergleich zu den aus dem Stand der Technik bekannten Eisenoxid-Pigmenten und lassen sich daher problemlos in Engoben und Glasuren einarbeiten. Die Glasuren können je nach gewünschtem Effekt matt bis glänzend sein, bzw. transparent bis opak. Gegenstand der Erfindung sind weiterhin auch Formulierungen, wie z.B. keramische Farben, Beschichtungen, Kacheln, Gießkeramiken, Sanitärkeramiken, Emaillen, Glasuren, Ton- , Glas- und Keramikwaren, die das erfindungsgemäße Interferenzpigment enthalten.

Die erfindungsgemäßen Interferenzpigmente sind weiterhin geeignet zur Herstellung von fließfähigen Pigmentpräparationen und Trockenpräparaten, insbesondere für Druckfarben und Lacke, vorzugsweise Autolacke, bestehend aus den erfindungsgemäßen Pigmenten, Bindemittel und optional ein oder mehreren Additiven.

Das erfindungsgemäße Interferenzpigment ist mit einer Vielzahl von Farbsystemen kompatibel, vorzugsweise aus dem Bereich der Lacke, Farben und Druckfarben. Für die Herstellung der Druckfarben für z. B. den Tiefdruck, Flexodruck, Offsetdruck, Offsetüberdrucklackierung, ist eine Vielzahl von Bindern, insbesondere wasserlösliche Typen, geeignet, wie sie z. B. von den Firmen BASF, Marabu, Pröll, Sericol, Hartmann, Gebr. Schmidt, Sicpa, Aarberg, Siegwerk, GSB-Wahl, Follmann, Ruco oder Coates Screen INKS GmbH vertrieben werden. Die Druckfarben können auf Wasserbasis oder Lösemittelbasis aufgebaut sein.

Es versteht sich von selbst, dass für die verschiedenen Anwendungszwecke das erfindungsgemäße Interferenzpigment auch vorteilhaft in Abmischung mit z. B.

- Metalleffektpigmenten, z. B. auf der Basis von Eisen- oder Aluminiumplättchen;
- Perlglanzpigmenten auf der Basis von metalloxidbeschichteten synthetischen Glimmer-Plättchen, natürlichen Glimmerplättchen, Glas-Plättchen, $Al_2O_3$-Plättchen, $Fe_2O_3$-Plättchen oder $SiO_2$-Plättchen;
- Absorptionspigmenten;
- goniochromatischen Pigmenten;
- Mehrschichtpigmenten (enthaltend vorzugsweise 2, 3, 4, 5 oder 7 Schichten) auf der Basis von metalloxidbeschichteten synthetischen Glimmer-Plättchen, natürlichen Glimmerplättchen, Glas-Plättchen, $Al_2O_3$-Plättchen, $Fe_2O_3$-Plättchen oder $SiO_2$-Plättchen;
- organischen Farbstoffen;
- organischen Pigmenten;
- anorganischen Pigmenten, wie z.B. transparenten und deckenden Weiß-, Bunt- und Schwarzpigmenten; insbesondere temperaturstabile keramische Pigmente;
- plättchenförmigen Eisenoxiden;
- Ruß;
- keramischen Farbkörpern;
- funktionellen Pigmenten, z.B. IR-reflektierende oder elektrisch leitfähige Pigmente eingesetzt werden kann.

In einer bevorzugten Ausführungsform wird das erfindungsgemäße Interferenzpigment vorteilhaft mit organischen und anorganischen Farbpigmenten und Farbstoffen, natürlichen oder synthetischen Ursprungs, wie z.B. Karminrot, Chromoxid, Ultramarin, sphärischen $SiO_2$- oder $TiO_2$-Pigmenten, gemischt. Das Mischungsverhältnis ist abhängig vom Anwendungsmedium und dem Effekt, der erzielt werden soll.

Das erfindungsgemäße Interferenzpigment kann in jedem Verhältnis mit handelsüblichen Pigmenten und/oder weiteren handelsüblichen Füllstoffen gemischt werden.

Für den Einsatz als Karminrot-Ersatz in veganen Kosmetik-Formulierungen sei hier explizit die Abmischung mit Silberweiß-Interferenzpigmenten, Titandioxid oder anderen Weißpigmenten erwähnt. Durch die verschiedenen Mischungsverhältnisse lassen sich die gewünschten Farbschattierungen in einem breiten Pinkbereich abdecken.

Als handelsübliche Füllstoffe sind z.B. zu nennen natürlicher und synthetischer Glimmer, Glasbeads, Glaspulver, Nylon Powder, reine oder gefüllte Melaminharze, Talkum, Gläser, Kaolin, Oxide oder Hydroxide von Aluminium, Magnesium, Calcium, Zink, BiOCl, Bariumsulfat, Calciumsulfat, Calciumcarbonat, Magnesiumcarbonat, Kohlenstoff, Bornitrid sowie physikalische oder chemische Kombinationen dieser Stoffe. Bezüglich der Partikelform des Füllstoffes gibt es keine Einschränkungen. Sie kann den Anforderungen gemäß unregelmäßig geformt, plättchenförmig, sphärisch oder nadelförmig, kristallin oder amorph sein.

Das erfindungsgemäße Interferenzpigment ist insbesondere geeignet für die dekorative Kosmetik und für Personal Care Anwendungen, wie z.B. Nagellacke, Lippenstifte, Lipgloss, Rouge, Presspuder, Gele, Lotionen, Seifen, Zahnpasta, Body Lotions, Emulsionen, Seifen, Shampoos, BB Cremes, CC Cremes, Make Up, Foundations, (Volumen) Mascara, Haar-, Wimpern- und Augenbrauenprodukte, Sonnenschutz, Prä-Sun- und After-Sun-Präparate, Make-ups, Body Lotions, Badegele, Seifen, Badesalze, Zahnpasta, Haargele, Presspuder, lose Puder, etc.

In der dekorativen Kosmetik wird das erfindungsgemäße Interferenzpigment vorzugsweise in Konzentrationen von 0,5 - 25 Gew.%, insbesondere 1 - 20 Gew.%, und ganz besonders bevorzugt von 1 - 10 Gew.%, bezogen auf die Formulierung eingesetzt. Bei den kosmetischen Formulierungen für Personal Care Anwendungen wird das erfindungsgemäße Interferenzpigment vorzugsweise in Konzentrationen von 0,1 - 5 Gew.%, und ganz besonders bevorzugt von 0,5 - 4 Gew.%, bezogen auf die Formulierung eingesetzt.

Selbstverständlich können die erfindungsgemäßen Interferenzpigmente auch mit jeder Art von kosmetischen Roh- und Hilfsstoffen kombiniert werden. Dazu gehören u.a. Öle, Fette, Wachse, Filmbildner, Tenside, Antioxidantien, wie z.B. Vitamin C oder Vitamin E, Stabilisatoren, Geruchsverstärker, Silikonöle, Emulgatoren, Lösemittel wie z.B. Ethanol, oder Ethylacetat oder Butylacetat, Konservierungsmittel und allgemein anwendungstechnische Eigenschaften bestimmende Hilfsstoffe, wie z.B. Verdicker und rheologische Zusatzstoffe wie etwa Bentonite, Hektorite, Siliciumdioxide, Ca-Silicate, Gelatinen, hochmolekulare Kohlenhydrate und/oder oberflächenaktive Hilfsmittel, etc.

Die das erfindungsgemäße Interferenzpigment enthaltende Formulierung kann dem lipophilen, hydrophilen oder hydrophoben Typ angehören. Bei heterogenen Formulierungen mit diskreten wässrigen und nichtwässrigen Phasen kann das erfindungsgemäße Interferenzpigment in jeweils nur einer der beiden Phasen enthalten oder auch über beide Phasen verteilt sein.

Die pH-Werte der Formulierungen können zwischen 1 und 14, bevorzugt zwischen 2 und 11 und besonders bevorzugt zwischen 4 und 10 liegen.

Den Konzentrationen des erfindungsgemäßen Interferenzpigments in der Formulierung sind keine Grenzen gesetzt. Sie können - je nach Anwendungsfall - zwischen 0,001 (rinse-off-Produkte, z.B. Duschgele) - und 60 % liegen. Das erfindungsgemäße Interferenzpigment kann weiterhin auch mit kosmetischen Wirkstoffen kombiniert werden. Geeignete Wirkstoffe sind z.B. Insect Repellents, anorganische UV-Filter, wie z.B. $TiO_2$, UV A/BC-Schutzfilter (z.B. OMC, B3, MBC), auch in verkapselter Form, Anti-Ageing-Wirkstoffe, Vitamine und deren Derivate (z.B. Vitamin A, C, E, etc.), Selbstbräuner (z.B. DHA, Erythrulose, u.a.) sowie weitere kosmetische Wirkstoffe, wie z.B. Bisabolol, LPO, Ectoin, Emblica, Allantoin, Bioflavanoide und deren Derivate.

Organische UV-Filter werden in der Regel in einer Menge von 0,5 bis 10 Gewichtsprozent, vorzugsweise 1 - 8 %, anorganische Filter von 0,1 bis 30% in kosmetische Formulierungen eingearbeitet.

Die Formulierungen können darüber hinaus weitere übliche hautschonende oder hautpflegende Wirkstoffe enthalten, wie z.B. Aloe Vera, Avocadoöl, Coenzym Q10, Grüner Tee Extrakt und auch Wirkstoffkomplexe. Besonders bevorzugte Wirkstoffe sind Pyrimidincarbonsäuren und/oder Aryloxime.

Unter den kosmetischen Anwendungen ist insbesondere die Verwendung von Ectoin und Ectoin-Derivaten zur Pflege von gealterter, trockener oder gereizter Haut zu nennen. So wird in der europäischen Patentanmeldung EP-A-0 671 161 insbesondere beschrieben, dass Ectoin und Hydroxyectoin in kosmetischen Zubereitungen wie Pudern, Seifen, tensidhaltigen Reinigungsprodukten, Lippenstiften, Rouge, Make-Up, Pflegecremes und Sonnenschutzpräparaten eingesetzt werden.

Als Anwendungsform der kosmetischen Formulierungen seien z.B. genannt: Lösungen, Suspensionen, Emulsionen, PIT-Emulsionen, Pasten, Salben, Gele, Cremes, Lotionen, Puder, Seifen, tensidhaltige Reinigungspräparate, Öle, Aerosole und Sprays. Weitere Anwendungsformen sind z.B. Sticks, Shampoos und Duschbäder. Der Zubereitung können beliebige übliche Trägerstoffe, Hilfsstoffe und gegebenenfalls weitere Wirkstoffe zugesetzt werden.

Salben, Pasten, Cremes und Gele können die üblichen Trägerstoffe enthalten, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen Treibmittel, z.B. Chlorfluorkohlenwasserstoffe, Propan/Butan oder Dimethylether, enthalten.

Lösungen und Emulsionen können die üblichen Trägerstoffe wie Lösungsmittel, Lösungsvermittler und Emulgatoren, z.B. Wasser, Ethanol, Isopropanol, Ethylcarbonat, Ethylacetat, Benzylalkohol, Benzylbenzoat, Propylenglykol, 1,3-Butylglykol, Öle, insbesondere Baumwollsaatöl, Erdnussöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Suspensionen können die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Seifen können die üblichen Trägerstoffe wie Alkalisalze von Fettsäuren, Salze von Fettsäurehalbestern, Fettsäureeiweißhydrolysaten, Isothionate, Lanolin, Fettalkohol, Pflanzenöle, Pflanzenextrakte, Glycerin, Zucker oder Gemische dieser Stoffe enthalten.

Tensidhaltige Reinigungsprodukte können die üblichen Trägerstoffe wie Salze von Fettalkoholsulfaten, Fettalkoholethersulfaten, Sulfobernsteinsäurehalbestern, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können die üblichen Trägerstoffe wie synthetische Öle wie z.B. Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Die kosmetischen Zubereitungen können in verschiedenen Formen vorliegen. So können sie z. B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsion, beispielsweise vom Typ Waser-in-Öl-in-Wasser (W/O/W), ein Gel, einen festen Stift, eine Salbe oder auch ein Aerosol darstellen. Es ist auch vorteilhaft, Ectoine in verkapselter Form darzureichen, z. B. in Kollagenmatrices und anderen üblichen Verkapselungsmaterialien, z. B. als Celluloseverkapselungen, in Gelatine, Wachsmatrices oder liposomal verkapselt. Insbesondere Wachsmatrices wie sie in der DE-OS 43 08 282 beschrieben werden, haben sich als günstig herausgestellt. Bevorzugt werden Emulsionen. O/W-Emulsionen werden besonders bevorzugt. Emulsionen, W/O-Emulsionen und O/W-Emulsionen sind in üblicher Weise erhältlich.

Weitere Ausführungsformen stellen ölige Lotionen auf Basis von natürlichen oder synthetischen Ölen und Wachsen, Lanolin, Fettsäureestern, insbesondere Triglyceriden von Fettsäuren, oder ölig-alkoholische Lotionen auf Basis eines Niedrigalkohols, wie Ethanol, oder eines Glycerols, wie Propylenglykol, und/oder eines Polyols, wie Glycerin, und Ölen, Wachsen und Fettsäureestern, wie Triglyceriden von Fettsäuren, dar.

Feste Stifte bestehen aus natürlichen oder synthetischen Wachsen und Ölen, Fettalkoholen, Fettsäuren, Fettsäureestern, Lanolin und anderen Fettkörpern.

Ist eine Zubereitung als Aerosol konfektioniert, verwendet man in der Regel die üblichen Treibmittel, wie Alkane, Fluoralkane und Chlorfluoralkane.

Die kosmetische Zubereitung kann auch zum Schutz der Haare gegen photochemische Schäden verwendet werden, um Veränderungen von Farbnuancen, ein Entfärben oder Schäden mechanischer Art zu verhindern. In diesem Fall erfolgt geeignet eine Konfektionierung als Shampoo, Lotion, Gel oder Emulsion zum Ausspülen, wobei die jeweilige Zubereitung vor oder nach dem Shamponieren, vor oder nach dem Färben oder Entfärben bzw. vor oder nach der Dauerwelle aufgetragen wird. Es kann auch eine Zubereitung als Lotion oder Gel zum Frisieren und Behandeln, als Lotion oder Gel zum Bürsten oder Legen einer Wasserwelle, als Haarlack, Dauerwellenmittel, Färbe- oder Entfärbemittel der Haare gewählt werden. Die Zubereitung mit Lichtschutzeigenschaften kann Adjuvantien enthalten, wie Grenzflächen aktive Mittel, Verdickungsmittel, Polymere, weichmachende Mittel, Konservierungsmittel, Schaumstabilisatoren, Elektrolyte, organische Lösungsmittel, Silikonderivate, Öle, Wachse, Antifettmittel, Farbstoffe und/oder Pigmente, die das Mittel selbst oder die Haare färben oder andere für die Haarpflege üblicherweise verwendete Ingredienzien.

Gegenstand der vorliegenden Erfindung sind ebenfalls Formulierungen, insbesondere Formulierungen, die neben dem erfindungsgemäßen Interferenzpigment mindestens einen Bestandteil ausgewählt aus der Gruppe der Absorptionsmittel, Adstringenzien, antimikrobiellen Stoffe, Antioxidantien, Antiperspirantien, Antischaummitteln, Antischuppen-Wirkstoffe, Antistatika, Bindemittel, biologischen Zusatzstoffe, Bleichmittel, Chelatbildner, Desodorierungsmittel, Emollentien, Emulgatoren, Emulsionsstabilisatoren, Farbstoffe, Feuchthaltemittel, Filmbildner, Füllstoffe, Geruchsstoffe, Geschmacksstoffe, Insect Repellents, Konservierungsmittel, Korrosionsschutzmittel, kosmetischen Öle, Lösungsmittel, Oxidationsmittel, pflanzlichen Bestandteile, Puffersubstanzen, Reduktionsmittel, Tenside, Treibgase, Trübungsmittel, UV-Filter und UV-Absorber, Vergällungsmittel, Aloe Vera, Avocadoöl, Coenzym Q10, Grüner Tee Extrakt, Viskositätsreglern, Parfüm, anorganischen Pigmente, wie z.B. transparente oder deckende Weiß-, Bunt- und Schwarzpigmente, Metallpigmente, temperaturstabile keramische Pigmente, keramische Farbkörper, funktionelle Pigmente, wie z.B. IR-reflektierende Pigmente oder elektrisch leitfähige Pigmente, und Vitamine enthalten.

Das erfindungsgemäße Interferenzpigment kann zur Pigmentierung von Lebensmitteleinfärbungen, zur Veredlung von Lebensmitteln, in Lebensmittelüberzügen z. B. Masse-Einfärbung oder als farbiger Überzug, in Arzneimittelüberzügen, z.B. bei Dragees und Tabletten eingesetzt werden.

Die Einfärbung der Pharma- und Lebensmittelerzeugnisse erfolgt, indem das Interferenzpigment, in den gewünschten Mengenverhältnissen, dem einzufärbenden Erzeugnis in Mengen von 0,005 bis 15 Gew.%, vorzugsweise 0,01 bis 100 Gew.%, zugegeben wird.

Durch die Zumischung von für den Lebensmittelbereich zugelassenen, natürlichen bzw. naturidentischen Farbstoffen, organischen oder anorganischen Farbpigmenten oder färbenden natürlichen Frucht- und Pflanzenextrakten kann der Farbeffekt der erfindungsgemäßen Interferenzpigmente im Erzeugnis beeinflusst und gleichzeitig können neuartige changierende Farbeffekte erzielt werden.

Geeignete natürliche oder naturidentische Farbstoffe sind insbesondere E 101, E 104, E 110, E 124, E 131, E 132, E 140, E 141, E 151, E 160a. Weiterhin können auch andere Farbpigmente den erfindungsgemäßen Interferenzpigmenten beigemischt werden, wie z.B. E 171, E 172, E 153.

Der Anteil an Farbstoffen neben den erfindungsgemäßen Interferenzpigmenten bezogen auf das Lebensmittel- oder Pharmaprodukt liegt vorzugsweise im Bereich von 0,5 bis 25 Gew.%. Als Farbstoff können ebenfalls Frucht- und Pflanzenextrakte eingesetzt werden, wie z.B. Möhrensaft, Rote Beete-Saft, Holundersaft, Hibiskussaft, Paprikaextrakt, Aroniaextrakt.

Die Gesamtkonzentration aller Pigmente im zu pigmentierenden Erzeugnis sollte 50 Gew.% bezogen auf das Erzeugnis nicht übersteigen. Sie ist in der Regel abhängig vom konkreten Anwendungsfall.

Den Lebensmittel- und Pharmaprodukten können auch unterschiedliche Wirkstoffbeimischungen, wie z.B. Vitamine, Enzyme, Spurenelemente, Proteine, Kohlenhydrate, essenzielle Fette und / oder Mineralien zugesetzt werden, wobei die Gesamtmenge an Wirkstoffen bezogen auf das Lebensmittel bzw. Pharmaprodukt 25 Gew.% nicht übersteigen sollte. Vorzugsweise beträgt die Menge an Wirkstoffen bzw. Wirkstoffgemischen 0,01 - 20 Gew.% bezogen auf das Produkt.

Die Einfärbung der Produkte erfolgt, indem das Interferenzpigment allein oder in Kombination mit weiteren Pigmenten oder Färbemitteln direkt oder in Gegenwart von Wasser und/oder eines organischen Lösungsmittels in den gewünschten Mengenverhältnissen, gleichzeitig oder nacheinander, während oder nach ihrer Herstellung, vor oder nach der Formgebung (z.B. bei Extrusion, Pelletierung, Expandierung, Granulation, etc.) dem einzufärbenden Erzeugnis zugegeben wird. Eine Zumischung der erfindungsgemäßen Interferenzpigmente zu pulverförmigen bzw. losen Pulvern ist ebenfalls möglich.

Die erfindungsgemäßen Interferenzpigmente können allein oder im Pigmentgemisch auch zur Färbung der Lebensmittel- und Pharmaprodukte nach der Formgebung auf die Oberfläche appliziert werden. Hierbei wird das Interferenzpigment in der Regel mit einem Auftragsmedium vermischt und anschließend mit geeigneten Auftrags- und Sprühvorrichtungen auf das Produkt aufgetragen. Das Auftrags- bzw. Überzugsmittel sorgt dann für die entsprechende Anhaftung der Interferenzpigmente auf der Produktoberfläche. Diese wird dann entsprechend gefärbt.

Bei der Einarbeitung in die Produktmatrix selbst, liegt die Einsatzmenge der erfindungsgemäßen Interferenzpigmente vorzugsweise bei 0,5 - 40 Gew.%, insbesondere bei 1 - 30 Gew.%. Bei der Oberflächenfärbung von Lebensmittel- und Pharmaprodukten liegt der Einsatzbereich in der verwendeten Farb- bzw. Überzugslösung bei 0,1 - 25 Gew.%, insbesondere bei 1 - 15 Gew. %. Bei der Verwendung der erfindungsgemäßen Interferenzpigmente in pulverförmigen Produkten liegt der Einsatzbereich bei 0,05 - 50 Gew. %, insbesondere bei 2 - 10 Gew.%.

Die Überzugslösungen enthalten vorzugsweise Wasser oder organische Lösemittel, wie z.B. Ethanol oder Isopropanol. Als Filmbildner wird in den Überzugslösungen vorzugsweise ein Cellulosederivat, wie z.B. Hydroxypropylmethylcellulose, eingesetzt. Insbesondere bevorzugt sind Auftragslösungen mit Cellulosederivaten, die statt Wasser 5 - 80 Gew. % eines geeigneten organischen Lösemittels enthalten.

Gegenüber wässrigen Überzugslösungen besitzen die alkoholischen, bzw. alkoholischwässrigen, Cellulose-haltigen Auftragslösungen deutliche anwendungstechnische Vorteile:

- Einsatz von kühlerer Trocknungsluft während des Sprühauftrags
- Färbung von wärmeempfindlichen Produkten, wie z.B. vitaminhaltigen Lebensmitteln, mit den roten Interferenzpigmenten ist sehr gut möglich.

Als zur Einfärbung geeignete Produkte sind insbesondere zu nennen Überzüge auf allen Arten von Lebensmitteln, insbesondere pigmentierte Zucker- und Schellacküberzüge (alkoholische und wässrige), Überzüge mit Ölen und Wachsen, mit Gummi Arabicum und mit Cellulosearten (z.B. HPMC = Hydroxypropylmethylcellulose), mit Stärke- und Eiweißderivaten, Carragenen und sonstigen dem Fachmann bekannten zum Überziehen geeigneten Substanzen. Hierbei wird das erfindungsgemäße Interferenzpigment in der Regel mit dem Auftragsmedium vermischt und anschließend mit geeigneten Auftrags- und Sprühvorrichtungen, oder per Hand auf dem Lebensmittel- oder Pharmaprodukt aufgetragen. Das Auftrags- bzw. Überzugsmittel sorgt dann für die entsprechende Anhaftung der Pigmente auf der Oberfläche des Lebensmittels oder Pharmaprodukts. Diese wird dann entsprechend gefärbt. Die Auftrags- und Überzugslösungen enthalten vorzugsweise 0,1 - 20 Gew.%, insbesondere 2-15 Gew.% an Interferenzpigmenten.

Bevorzugte Trockenpulvermischungen für Coatings enthalten ein Cellulosederivat, wie z.B. Hydroxypropylmethylcellulose, Natriumcarboxymethylcellulose, ein Trennmittel, wie z.B. Lecithin oder Stearinsäure, einen Glanzverstärker, wie z.B. Maltodextrin und/oder Dextrose, und das erfindungsgemäße Interferenzpigment. Vorzugsweise enthalten derartige Trockenpulvermischungen das erfindungsgemäße Interferenzpigment in Mengen von 0,01 - 50 Gew.%, insbesondere 0,5 - 40 Gew.% bezogen auf die Pulvermischung. Diesen Trockenpulvermischungen können je nach Bedarf noch Farbstoffe, Geschmackstoffe, Vitamine, Süßstoffe, etc., zugesetzt werden.

Für die Einfärbung bzw. für das Coaten geeignete Produkte sind beispielsweise Zuckerwaren, Kuchendekorationen, Komprimate, Dragees, Kaugummis, Gummiwaren, Fondanterzeugnisse, Marzipanerzeugnisse, Füllmassen, Kakao- und Fettglasuren, Schokolade und schokoladenhaltige Produkte, Speiseeis, Cerealien, Snackprodukte, Überzugsmassen, Tortenspiegel, Zuckerstreuseln, Nonpareilles, Gelee- und Gelatinewaren, Bonbons, Lakritze, Zuckerguss, Zuckerwatte, Fett-, Zucker- und Crememassen, Puddings, Desserts, Tortenguss, Kaltschalen, Limonaden und Brausegetränke, Getränke mit stabilisierenden Additiven, wie z. B. Carboxymethylcellulose, gesäuerte und ungesäuerte Milchprodukte, wie z. B. Quark, Joghurt, Käse, Käserinden, Wursthüllen, etc.

Bei dragierten bzw. gecoateten Lebensmittel- und Pharmaprodukten ist die Kombination der erfindungsgemäßen Interferenzpigmente mit Aromastoffen (Pulver- bzw. Flüssigaromen), Säuren und/oder mit Süßstoffen, wie z.B. Aspartam, möglich um den optischen Effekt auch geschmacklich zu betonen.

Gegenstand der Erfindung sind somit alle Formulierungen aus dem Lebensmittel- und Pharmabereich enthaltend die erfindungsgemäßen Interferenzpigmente allein oder in Kombination mit weiteren Pigmenten/Pigmentgemischen oder Farbstoffen (natürliche bzw. naturidentische) als Färbemittel.

Ein weiteres großes Einsatzgebiet liegt im Pharma- und OTC-Bereich zur Einfärbung bzw. als Überzug von Tabletten, Gelatinekapseln, Dragees, Salben, Hustensaft, etc. In Kombination mit üblichen Coatings wie Polymethacrylaten und Cellulosearten, z.B. HPMC, kann das erfindungsgemäße Interferenzpigment vielfältig zur Einfärbung und Veredelung der Produkte eingesetzt werden.

Gegenstand der Erfindung ist weiterhin die Verwendung der erfindungsgemäßen Interferenzpigmente in Farben, Lacken, Druckfarben, Sicherheitsdruckfarben, Kunststoffen, keramischen Materialien, Gläsern, Glasuren, als Tracer, als Absorber zur Lasermarkierung von Kunststoffen und Papieren, in kosmetischen Formulierungen, zur Pigmentierung von Lebensmitteleinfärbungen, zur Veredlung von Lebensmitteln, z.B. Masse-Einfärbung oder als farbiger Überzug, in Arzneimittelüberzügen, z.B. bei Dragees und Tabletten. Weiterhin sind die erfindungsgemäßen Pigmente auch zur Herstellung von Pigmentpräparationen sowie zur Herstellung von Trockenpräparaten, wie z.B. Granulate, Chips, Pellets, Briketts, etc., geeignet. Die Trockenpräparate sind insbesondere für Lacke und Druckfarben geeignet.

Da das erfindungsgemäße Pigment RADAR-transparent ist, ist es ebenfalls für RADAR-Anwendungen geeignet, insbesondere im Automobil-Bereich.

Weiterhin kann das erfindungsgemäße Interferenzpigment für die Färbung von Solarzellen verwendet werden, z.B. durch die Applikation einen Lacks enthaltend das erfindungsgemäße Pigment auf dem Solarpanel.

Die nachfolgenden Beispiele sollen die Erfindung erläutern, ohne sie jedoch zu begrenzen. Sofern nicht anders angegeben beziehen sich Prozentzahlen auf Gewichtsprozent.

**Beispiele**

Beispiel 1

100 g $SiO_2$-Plättchen (mittlere Plättchendicke 365 nm) werden in 2 l demineralisiertem Wasser suspendiert und unter Rühren auf 85 °C erhitzt. Anschließend wird von einer $FeCl_3$-Lösung (Gehalt 7% Fe) so viel zudosiert, bis der gewünschte Abstellpunkt erreicht ist. Bei der Zudosierung wird der pH-Wert der Suspension durch Zugabe von 30 %-iger KOH auf 3,1 konstant gehalten.

Nach Beenden der $FeCl_3$-Zugabe wird der pH-Wert der Suspension mit 30%-iger KOH auf 6,5 gestellt. Anschließend wird die Pigmentvorstufe abfiltriert, gewaschen und bei 110 °C getrocknet. Zuletzt wird das Pigment bei 900 °C geglüht und gesiebt.

Das erhaltene, bläulich-rote Interferenzpigment zeigt folgende L,a,b-Werte:

Lackkarte über schwarz (gemessen mit einem Byk-mac I Spektralfotometer, Messwinkel 75°):

$$L* = 14,07$$

$$a* = 20,82$$

$$b* = 16,62$$

Lackkarte über schwarz (gemessen mit einem Byk-mac I Spektralfotometer, Messwinkel 15°):

$$L* = 72,03$$

$$a* = 74,63$$

$$b* = 21,35$$

Lackkarte über weiß (gemessen mit einem Byk-mac I Spektralfotometer Messwinkel 45°):

$$L^* = 27{,}58$$

$$a^* = 34{,}52$$

$$b^* = 29{,}50$$

Das erfindungsgemäße Pigment besitzt folgende PSD (Particle Size Distribution)-Werte (gemessen mit Malvern Mastersizer 3000):

$$D_{10} = 9{,}4 \ \mu m$$

$$D_{50} = 19{,}5 \ \mu m$$

$$D_{90} = 35{,}0 \ \mu m$$

Vergleichsbeispiel 1: Beispiel 1 aus der EP 1 681 318 A2

100 g Silika Flakes (mittlere Flake-Dicke: 365 nm) werden in 2 l vollentsalztem Wasser auf 75°C erhitzt. Unter Rühren werden 1120 ml $FeCl_3$-Lösung (entspricht 130 % $Fe_2O_3$) zugegeben. Der pH-Wert der Reaktionsmischung wird durch Zugabe von Natronlauge (30 %ig) konstant bei 3 gehalten. Nach Zugabe der $FeCl_3$-Lösung wird der pH-Wert mit Natronlauge (30 %ig) auf pH 5 angehoben. Das Produkt wird abfiltriert und mit vollentsalztem Wasser nachgewaschen. Nach Trocknung bei 110 °C wird bei 800 °C kalziniert.

Lackkarte über schwarz (gemessen mit einem Byk-mac I Spektralfotometer, Messwinkel 75°):

$$L^* = 14{,}62$$

$$a^* = 21{,}85$$

$$b^* = 18{,}67$$

Lackkarte über schwarz (gemessen mit einem Byk-mac I Spektralfotometer, Messwinkel 15°):

$$L^* = 74{,}97$$

$$a^* = 74{,}57$$

$$b^* = 41{,}77$$

Lackkarte über weiß (gemessen mit einem Byk-mac I Spektralfotometer, Messwinkel 45°):

$$L^* = 28{,}69$$

$$a^* = 35{,}67$$

$$b^* = 34{,}18$$

Das Pigment gemäß Vergleichsbeispiel 1 besitzt folgende PSD (Particle Size Distribution)-Werte (gemessen mit Malvern Mastersizer 3000):

$$D_{10} = 8{,}9 \ \mu m$$

$$D_{50} = 18,9 \ \mu m$$

$$D_{90} = 34,9 \ \mu m$$

Das Pigment aus Beispiel 1 ist insbesondere wegen seiner Farbreinheit und Luminanz für kosmetische Anwendungen und Lebensmittelanwendungen geeignet.

Im direkten Vergleich des b*-Wertes (gemessen mit einem Byk-mac I Spektralfotometer) - als Maß für den Blauanteil (je niedriger der b*-Wert, desto höher der Blauanteil) wird unter den 3 typischen Beobachtungswinkel für Perlglanzpigmente deutlich, dass das Pigment gemäß Beispiel 1 unter allen 3 Betrachtungswinkeln signifikant blauer ist als das Pigment aus Vergleichsbeispiel 1:

| b*-Wert | Lackkarte über schwarz 75° | Lackkarte über schwarz 15° | Lackkarte über weiß 45° |
|---|---|---|---|
| Beispiel 1 | 16,62 | 21,35 | 29,50 |
| Vergleichsbeispiel 1 | 18,67 | 41,77 | 34,18 |

Beispiel 2 - Nachbeschichtung von Pigment gemäß Beispiel 1

Eine 2 Gew.-% wässrige Lösung mit 2,55 g Natriumhypophosphat ($NaH_2PO_2*H_2O$) wird bei Raumtemperatur unter Rühren zu einer 5 Gew.-% wässrigen Lösung mit 3,90 g Zirkoniumoxychlorid ($ZrOCl_2*8H_2O$) so langsam zugegeben, dass kein weißer Niederschlag entsteht. Zu der so erhaltenen klaren Lösung werden 4,1 g einer 35 Gew.-% Hydrochloridsäure zugegeben, wodurch eine Mischlösung aus Zirkoniumoxychlorid und Natriumhypophosphat entsteht.

Das Pigment gemäß Beispiel 1 wird in 1000 ml entionisiertem Wasser suspendiert. Zu der Suspension, die bei etwa 70 °C gehalten und mit 10 % HCl auf pH 2,5 eingestellt wird, werden 2,95 g Cer(III)-chlorid ($CeCl_3*7H_2O$) zugesetzt. Die Lösung aus Zirkoniumoxychlorid und Natriumhypophosphat wird 60 Minuten lang zugegeben, wobei der pH-Wert bei 2,5 mit 10 % NaOH konstant gehalten wird. Anschließend wird der pH-Wert innerhalb von 60 Minuten durch Zugabe von 10 % NaOH auf 7,0 erhöht. 1,5 g 3-Aminopropyl-trimethoxysilan (CAS-Nr. 13822-56-5), 1,5 g 3-Glycidyloxypropyltrimethoxysilan (CAS-Nr. 2530-83-8) und 0,5 g n-Hexyltrimethoxysilan (CAS-Nr. 3069-19-0) werden nachträglich jeweils 15 Minuten lang der Suspension zugegeben, wobei der pH-Wert bei 7,0 mit 10 % HCl oder 10 % NaOH gehalten wird. Das so oberflächenbehandelte Pigment wird abfiltriert, mit Wasser gewaschen, bei 140 °C getrocknet und gesiebt (325 Mesh).

Das Effektpigment weist eine sehr gute Wasser- und Witterungsbeständigkeit auf.

**Anwendungsbeispiele**

Beispiel A 1: Lippenstift

| Rohstoff | Bezugsquelle | INCI EU | [%] |
|---|---|---|---|
| Phase A | | | |
| Interferenzpigment gemäß Beispiel 1 | (1) | CI 77491, SILICA | 11,60 |
| RonaFlair® White Sapphire | (1) | SYNTHETIC SAPPHIRE | 10,00 |
| Covapate Unired NA 3781 | (2) | RICINUS COMMNUNIS SEED OIL, CI 77491 | 1,00 |
| Phase B | | | |
| Oxynex® K liquid | (1) | PEG-8, TOCOPHEROL, ASCORBYL PALMITATE ASCORBIC ACID, CITRIC ACID | 0,05 |
| Mirasil® Stearyl DM | (3) | STEARYL DIMETHICONE | 2,00 |
| Kaboguard® 50AMP-G | (4) | ACRYLATES/ETHYLHEXYL ACRYLATE COPOLYMER, AQUA, AMINOMETHYL PROPANOL | 3,40 |
| Bentone Gel ISD V | (5) | ISODODECANE, DISTEARDIMONIUM HECTORITE, PROPYLENE CARBONATE | 3,50 |

(fortgesetzt)

| Phase B | | | |
|---|---|---|---|
| JBC WAX 200 | (6) | SYNTHETIC CANDELILLA WAX | 6,50 |
| Lanol 99 | (7) | ISONONYL ISONONANOATE | 9,40 |
| MAKIGREEN™ VELVET WAX | (8) | ORYZA SATIVA, ORYZA SATIVA BRAN OIL, HELIANTHUS ANNUUS, RHUS SUCCEDANEA FRUIT WAX | 14,00 |
| Cetiol® Ultimate | (9) | UNDECANE, TRIDECANE | 18,00 |
| PURESIL™ TMS DM 30 | (3) | DIMETHICONE/TRI-METHYLSILOXY SILICATE | add 100 |
| Phase C | | | |
| FRAGRANCE | | PARFUM | q.s. |

Herstellung

Die Bestandteile der Phase B werden auf 80 °C erhitzt und aufgeschmolzen. Die Rohstoffe der Phase A werden zugegeben und alles gut durchrührt. Die Lippenstiftmasse wird dann in der auf 75 °C temperierten Gießapparatur gerührt bis sie keine Luftblasen mehr enthält. Die homogene Schmelze wird in die auf 55 °C vorgewärmten Gießform gegossen. Anschließend kühlt man die Formen ab (ca. 1 Stunde), entfernt die Gießlinge kalt und setzt sie in die Lippenstifthülsen ein. Anschließend werden die Lippenstifte kurz abgeflammt.

Hersteller

(1) Merck KGaA / EMD Performance Materials Corp.
(2) Sensient Cosmetic Technologies
(3) Elkem
(4) Kobo Products
(5) Elementis Specialities
(6) Alpha Quimica
(7) Seppic
(8) PIC Quimica
(9) BASF AG

Beispiel A2: Gesichtswasser (Micellar Water)

| Rohstoff | Bezugsquelle | INCI EU | [%] |
|---|---|---|---|
| Phase A | | | |
| Glycerol 85% | (1) | GLYCERIN, AQUA | 5,00 |
| Solagum™ AX | (2) | ACACIA SENEGALGUM, XANTHAN GUM | 0,80 |
| Water, demineralized | | AQUA | add 100,00 |
| Phase B | | | |
| Schercoteric™ C-2 50LT surfactant | (3) | DISODIUM COCOAMPHODIACETATE | 1,00 |
| Pluracare® L 64 G | (4) | STEARYL DIMETHICONE | 0,20 |
| Phase C | | | |
| RonaCare® RenouMer Cos | (1) | AQUA, POLYSIPHONIA ELONGATA EXTRACT, SODIUM BENZOATE, CITRIC ACID | 1,00 |

(fortgesetzt)

| Phase D | | | |
|---|---|---|---|
| RonaFlair® White Sapphire | (1) | SYNTHETIC SAPPHIRE | 3,00 |
| Interferenzpigment gemäß Beispiel 1 | (1) | CI 77491, Silica | 0,50 |
| Sodium Hydroxide, 10% | (1) | AQUA, SODIUM HYDROXIDE | 0,20 |
| Phase E | | | |
| Lipocol® HCO-40 | (5) | PARFUM | 0,05 |
| FRAGRANCE | (1) | PEG-40 HYDROGENATED CASTOR OIL | q.s. |
| Preservatives | (1) | PRESERVATIVES | q.s. |

Herstellung

Unter starkem Rühren wird die Phase B zu Phase A gegeben. Nach der Homogenisierung wird die Phase C zugegeben und der pH auf 5.0-5.5 eingestellt. Phase D wird zu der vorgemischten Phase E langsam zugegeben und alles wird unter Rühren mit der Phase A/B/C gemischt.

Hersteller

(1) Merck KGaA / EMD Performance Materials Corp.
(2) Seppic
(3) Lubrizol
(4) BASF AG
(5) Vantage Personal Care

Beispiel A3: Lidschatten

| Rohstoff | Bezugsquelle | INCI EU | [%] |
|---|---|---|---|
| Phase A | | | |
| Ronastar® Blue Lights | (1) | SYNTHETIC SAPPHIRE, CI 77891, SILICA, TIN OXIDE | 37,00 |
| Interferenzpigment gemäß Beispiel 1 | (1) | | 12,00 |
| Phase B | | | |
| RonaFlair® Mica M | (1) | MICA | 20,60 |
| RonaFlair® Soft Sphere | (1) | SYNTHETIC FLUORPHLOGOPITE, SILICA | 10,00 |
| Mq-Stearate | (2) | MAGNESIUM STEARATE | 2,00 |
| DOWSIL™ 9701 Cosmetic Powder | (3) | DIMETHICONE/VINYL DIMETHICONE CROSSPOLYMER, SILICA | 1,40 |
| Phase C | | | |
| Squalane | (2) | ISONONYL ISONONANOATE | add 100,00 |
| Eutanol G | (4) | ORYZA SATIVA, ORYZA SATIVA BRAN OIL, HELIANTHUS ANNUUS, RHUS SUCCEDANEA FRUIT WAX | 4,25 |

(fortgesetzt)

| Phase C | | | |
|---|---|---|---|
| KF-96A-6cs | (5) | UNDECANE, TRIDECANE | 14,25 |
| Preservative | | DIMETHICONE/TRI-METHYLSILOXY SILICATE | q.s. |

Herstellung

Mischen der Komponenten der Phase B und Zugabe der Phase A. Unter Rühren Zugabe des Gemisches der Phase C. Das Pulver bei 40-50 bar pressen.

Hersteller

(1) Merck KGaA / EMD Performance Materials Corp.
(2) China Chemical Reagent
(3) Dow Corning
(4) BASF AG
(5) Shin Etsu Silicones

Beispiel A4: Lip Gloss

| Rohstoff | Bezugsquelle | INCI EU | [%] |
|---|---|---|---|
| Phase A | | | |
| Interferenzpigment | | | 1,00 |
| Timiron® Supersilk MP-1005 | (1) | MICA, CI 77491 | 2,50 |
| Colorona® Fine Gold MP-20 | (1) | CI 77891, MICA, CI 77491 | 5,00 |
| Covanol White ON 9788 | (2) | CI 77891, OCTYLDODECANOL, STEARIC ACID, ALUMINUM HYDROXIDE, POLYHYDROXYSTEARIC ACID | 0,50 |
| Phase B | | | |
| Eusolex® 2292 | (1) | MICA | 7,50 |
| Eusolex® OS | (1) | SYNTHETIC FLUORPHLOGOPITE, SILICA | 5,00 |
| RonaCare® Bisabolol nat. | (1) | MAGNESIUM STEARATE | 0,40 |
| Oxynex® ST liquid | (1) | DIMETHYLHEXYL SYRINGYLIDENEMALONATE, CAPRYLIC/CAPRIC TRIGLYCERIDE | 0,30 |
| Oxynex® K liquid | (1) | PEG-8, TOCOPHEROL, ASCORBYL PALMITATE ASCORBIC ACID, CITRIC ACID | 0,50 |
| Finsolv TPP | (3) | C12-15 ALKYL BENZOATE, DIPROPYLENE GLYCOL DIBENZOATE | 3,00 |
| Myritol 312 | | | 3,00 |
| Eutanol G | | | 4,00 |
| Cetiol CC | | | 4,60 |
| Miglyol® Gel 840 B | | | 10,00 |
| Softisan 649 | | | add 100,00 |

(fortgesetzt)

| Phase B | | | |
|---|---|---|---|
| Preservative | | | q.s. |
| Phase C | | | |
| Aerosil® R 812 S | (8) | SILICA SILYLATE | 2,00 |

Herstellung

Komponente der Phase B unter Rühren mischen. Zugabe unter Rühren der Komponenten der Phase A und anschließend der Phase C. Zuletzt die Masse in ein geeignetes Behältnis abfüllen.

Hersteller

(1) Merck KGaA / EMD Performance Materials Corp.
(2) Sensient Cosmetic Technologies
(3) Innospec
(4) BASF AG
(5) Croda
(6) Sasol Germany GmbH
(7) IOI Oleo GmbH
(8) Evonik Nutrition & Care GmbH

Beispiel A5: Nail Polish

| Rohstoff | Bezugsquelle | INCI EU | [%] |
|---|---|---|---|
| Interferenzpigment nach Beispiel 1 | | | 4,00 |
| Nagellack-Base 12897 | (1) | ETHYL ACETATE, BUTYL ACETATE, NITROCELLULOSE, PHTHALIC ANHYDRIDE/TRIMELLITIC ANHYDRIDE/GLYCOLS COPOLYMER, ACETYL TRIBUTYL CITRATE, ISOPROPYL ALCOHOL, STEARALKONIUM HECTORITE, ADIPIC ACID/NEOPENTYL GLYCOL/TRIMELLITIC ANHYDRIDE COPOLYMER | 96,00 |

Herstellung

Das Interferenzpigment wird mit der Lackbase gut vermischt (1000 U/min) für 10 Minuten.

Hersteller

(1) International Lacquers S. A.

Beispiel A6: Lip Gloss

| Rohstoff | Bezugsquelle | INCI EU | [%] |
|---|---|---|---|
| Phase A | | | |
| Interferenzpigment gemäß Beispiel 1 | | | 4,00 |
| Phase B | | | |
| VPT162 - Vegetable Petrolatum Transparent 162 | (2) | RICINUS COMMUNIS SEED OIL, HYDROGENATED CASTOR OIL, COPERNICIA CERIFERA CERA, CERA ALBA | 40,00 |
| Kahlresin 5723 MB | (1) | GLYCERYL ROSINATE, OCTYLDODECANOL | 30,00 |
| Castor Oil, pressed | (2) | RICINUS COMMUNIS SEED OIL | 15,50 |
| Refined Aprikosenöl | (3) | PRUNUS ARMENIACA KERNEL OIL | 10,00 |
| Phase C | | | |
| All-rac-alpha-Tocopherol | (1) | TOCOPHEROL | 0,50 |

Herstellung

Phase B auf 70 °C erhitzen und unter Rühren homogenisieren. Unter Rühren langsam auf 50 °C abkühlen lassen und das Interferenzpigment (Phase A) hinzugeben. Weiter auf 35 °C abkühlen lassen und Phase C hinzugeben und anschließend in geeignete Behälter abfüllen.

Hersteller

(1) Merck KGaA / EMD Performance Materials Corp.
(2) DKSH GmbH
(3) Kahl GmbH & Co. KG
(4) IES Labo

Beispiel A7: Rouge

| Rohstoff | Bezugsquelle | INCI EU | [%] |
|---|---|---|---|
| Phase A | | | |
| Interferenzpigment gemäß Beispiel 1 | | | 7,00 |
| Timiron® Glam Silver | (1) | Alumina, CI 77891, TIN OXIDE | 3,00 |
| RonaFlair® Satin | (1) | ILLITE | 20,00 |
| Phase B | | | |
| Oxynex® L-CV Liquid | (1) | ALCOHOL, TOCOPHEROL, CAPRYLIC/CAPRIC TRIGLYCERIDE, ASCORBYL PALMITATE, ASCORBIC ACID, CITRIC ACID | 0,30 |
| Organic Jojoba Oil | (2) | SIMMONDSIA CHINENSIS SEED OIL | 2,00 |
| Crodamol™ GTCC MBAL-LQ-(MV) | (3) | CAPRYLIC/CAPRIC TRIGLYCERIDE | 7,00 |

(fortgesetzt)

| | | | |
|---|---|---|---|
| **Phase B** | | | |
| Eutanol G | (4) | OCTYLDODECANOL | 12,00 |
| Cetiol OE | (4) | DICAPRYLYL ETHER | 16,00 |
| **Phase C** | | | |
| Bentone Gel | (5) | PROPYLENE CARBONATE, C12-15 ALKYL BENZOATE, STEARALKONIUM HECTORITE | 32,70 |

Herstellung

Die Bestandteile von Phase A und von Phase separat mischen. Phase C zu Phase B geben und mischen. Phase A zu dem Gemisch aus Phase B/C geben und alles mit dem Ultra-Turrax bei 8000 U/min homogenisieren.

Hersteller

(1) Merck KGaA / EMD Performance Materials Corp.
(2) IES Labo
(3) Croda
(4) BASF AG
(5) Nordmann, Rassmann GmbH

Beispiel A8: Lippenstift

| Rohstoff | Bezugsquelle | INCI EU | [%] |
|---|---|---|---|
| **Phase A** | | | |
| Interferenzpigment gemäß Beispiel 1 | | | 18,00 |
| RonaFlair® LDP White | (1) | SODIUM POTASSIUM ALUMINUM SILICATE, CI 77891, SILICA | 5,00 |
| **Phase B** | | | |
| RonaCare® Bisabolol nat. | (1) | BISABOLOL | 0,50 |
| RonaCare® Poppy SE | (1) | CAPRYLIC/CAPRIC TRIGLCERIDE, PAPAVER RHOEAS EXTRACT, TOCOPHEROL | 0,50 |
| Oxynex® L-CV Liquid | (1) | ALCOHOL, TOCOPHEROL, CAPRYLIC/CAPRIC TRIGLYCERIDE, ASCORBYL PALMITATE, ASCORBIC ACID, CITRIC ACID | 0,50 |
| Cetiol SB 45 | (2) | BUTYROSPERMUM PARKII BUTTER | 3,00 |
| Orange Peel Wax | (3) | CITRUS AURANTIUM DULCIS (ORANGE) PEEL WAX | 3,00 |
| Sunflower Wax | (3) | HELIANTHUS ANNUUS SEED CERA | 5,00 |
| Beeswax white, organic | (4) | CERA ALBA | 12,00 |
| Myritol® 318 | (2) | CAPRYLIC/CAPRIC TRIGLCERIDE | |

(fortgesetzt)

| Phase B | | | |
|---|---|---|---|
| Organic Jojoba Oil | (5) | SIMMONDSIA CHINENSIS SEED OIL | 18,00 |
| Castor Oil, pressed, organic | (4) | RICINUS COMMUNIS SEED OIL | 20,00 |

Herstellung

Die Bestandteile der Phase B werden auf 80-85 °C erhitzt und aufgeschmolzen. Die Rohstoffe der Phase A werden zugegeben und alles gut durchmischt. Die Lippenstiftmasse wird dann in der auf 80 °C temperierten Gießapparatur gerührt bis sie keine Luftblasen mehr enthält. Die homogene Schmelze wird in die auf 55 °C vorgewärmten Gießform gegossen. Anschließend kühlt man die Formen ab (ca. 1 Stunde), entfernt die Gießlinge kalt und setzt sie in die Lippenstifthülsen ein. Zuletzt werden die Lippenstifte kurz abgeflammt.

Hersteller

(1) Merck KGaA / EMD Performance Materials Corp.
(2) BASF AG
(3) Kloster Keunen Holland BV
(4) Henry Lamotte Oils GmbH
(5) IES Labo

Beispiel A9: Lip Gloss

| Rohstoff | Bezugsquelle | INCI EU | [%] |
|---|---|---|---|
| Phase A | | | |
| Interferenzpigment gemäß Beispiel 1 | | | 1,00 |
| RonaCare® Poppy SE | (1) | CAPRYLIC/CAPRIC TRIGLCERIDE, PAPAVER RHOEAS EXTRACT, TOCOPHEROL | 0,50 |
| RonaCare® Bisabolol nat. | (1) | BISABOLOL | 0,10 |
| Phase B | | | |
| Oxynex® ST Liquid | (1) | DIETHYLHEXYL SYRINGYLIDENEMAL ONATE, CAPRYLIC/CAPRIC TRIGLYCERIDE | 0,10 |
| El Shea Butter | (2) | SHEA BUTTER | 3,00 |
| LexFeel Shine | (3) | PROPYLENE GLYCOL DIBENZOATE | 4,00 |
| Beeswax | (4) | CERA ALBA | 4,00 |
| Myritol 318 | (5) | CAPRYLIC/CPRIC TRIGLYCERIDE | 5,40 |
| Crystal Crown® Castor Oil | (2) | RICINUS COMMUNIS (CASTOR) SEED OIL | 8,90 |
| Cetiol® 868 | (5) | ETHYLHEXYL STEARATE | 10,00 |

(fortgesetzt)

| Phase B | | | |
|---|---|---|---|
| Versagel® ME 750 | (6) | HYDROGENATED, POLYISOBUTENE, ETHYLENE/-PROPYLE NE/STYRENE COPOLYMER, BUTYLENE/ETHYLEN E/STYRENE COPLYMER; BHT | 24,00 |
| Indopol H-100 | (7) | POLYBUTENE | 30,00 |

Herstellung

Phase B unter Rühren auf 90-95 °C erhitzen bis sich eine klare Emulsion bildet. Temperatur auf 55-60 °C runter kühlen und unter Rühren Zugabe von Phase A. Weiter auf 45-50 °C abkühlen und anschließend in geeignete Behälter abfüllen.

Hersteller

(1) Merck KGaA / EMD Performance Materials Corp.
(2) Essential Ingredients
(3) INOLEX Chemical Company
(4) Strahl & Pitsch
(5) BASF AG
(6) Penreco
(7) Ineos Oligomers

Beispiel A 10: Herstellung von Hartkaramellen

| Rohstoff | % | Bezugsquellen: |
|---|---|---|
| Zucker | 41 % | Fa. Südzucker |
| Wasser | 17,118 % | |
| Glucosesirup | 41 % C* Sweet | Fa. Cerestar, Krefeld |
| Interferenzpigment gemäß Beispiel 1 | 0,082 % (0,1 % bezogen auf die Gießmasse) | Fa. Merck KGaA, Darmstadt |
| E 104 1 : 100 verd. | 0,4 % Sikovit | Fa. BASF, Ludwigshafen |
| Aroma | 0,4 % (Banane 9/030388) | Fa. Dragaco, Holzminden |

Der Zucker wird mit dem Wasser auf 100 °C erhitzt und danach mit Glucosesirup versetzt. Die Lösung wird anschließend auf 145 °C erhitzt. Nach Zugabe des Interferenzpigments, der Farblösung und dem Aroma wird die Karamellösung mit einem Gießtrichter in gefettete Formen gegossen. Zuletzt lässt man zwei Stunden abkühlen. Das Interferenzpigment kann sowohl mit dem Zucker vermischt werden als auch mit dem Glukosesirup vermischt zugegeben werden. Diese Variante enthält keine Säure, da hierdurch die Karamelisation zu stark würde.

Beispiel A 11: Herstellung von Gelatineartikeln

| Rohstoff | % | Bezugsquellen: |
|---|---|---|
| Wasser | 10,48 % | |
| Zucker | 31,45 % | Fa. Südzucker |
| Glukosesirup | 31,45 % C*Sweet | Fa. Cerestar, Krefeld |

(fortgesetzt)

| Rohstoff | % | Bezugsquellen: |
|---|---|---|
| Interferenzpigment gemäß Beispiel 1 | 0,38 % (0,4% bezogen auf die Gießmasse) | Fa. Merck KGaA, Darmstadt |
| Zitronensäure 1:1 verd. | 2,51 % | Fa. Merck KGaA, Darmstadt |
| Gelatine | 7,86 % 260 Bloom | Fa. DGF, Eberbach |
| Wasser | 15,748 % | |
| Aroma | 0,122 % (schwarze Johannisbeere 9/695750) | Fa. Dragoco, Holzminden |

Zunächst wird die Gelatine mit der doppelten Menge an Wasser bei 60 °C eingeweicht. Zucker und Wasser werden auf 100 °C erhitzt, dann wird der Glucosesirup zugegeben. Man erhitzt weiter auf 120 °C und lässt dann auf ca. 85 °C abkühlen. Das Interferenzpigment, die Zitronensäure, das Aroma und die Gelatinelösung werden untergerührt, und das entlüftete Gelatinegemisch wird mit dem Gießtrichter in gefettete Formen abgefüllt. Das Produkt lässt man ca. 16 Stunden abkühlen.

Weitere Ausführungsformen:

- Das bläulich-rote Interferenzpigment kann hierbei wieder direkt mit dem Zucker vermischt werden oder mit dem Glukosesirup eingebracht werden.
- Anstelle des Gießens in Formen kann auch die traditionelle Technik mit Negativformen in Formpuder zur Herstellung von Gelatineartikeln hierbei verwendet werden.

Beispiel A 12: Coating von Tabletten

a) Einwaage 1 kg weiße Tabletten d=8mm, G=200mg

| Lösung für das Filmcoating: | | |
|---|---|---|
| 6% | Sepifilm Lp10 (Gemisch aus Hydroxypropylmethylcellulose, Stearinsäure und mikrokristalliner Cellulose) | Fa. Seppic |
| 5 % | Interferenzpigment gemäß Beispiel 1 | Fa. Merck KGaA, Darmstadt |
| 89 % | Wasser | |
| Gesamtauftragsmenge: 200 g Dies entspricht 1,2 mg Polymer/cm$^2$ Tablettenoberfläche | | |

Herstellung der Film-Coating-Lösung:

- Das Interferenzpigment wird in Wasser eingerührt. Anschließend fügt man gegebenenfalls zusätzliche Farbstoffe hinzu. Schließlich streut man den Filmbildner (HPMC) in die Suspension ein. Durch die ansteigende Viskosität bedingt, muss auch die Rührgeschwindigkeit dementsprechend erhöht werden. Nach ca. 40-60 Minuten ist die HPMC vollständig gelöst und die Lösung kann nun auf die Tabletten aufgesprüht werden.
- Der Sprühauftrag erfolgt mittels gängigem Standard-Coating-Verfahren.

Beispiel A13: Keramik

1) Herstellen der Druckpaste:

Zur Herstellung von feinen Farbrastern und reliefartigen Drucken auf keramischen Unterlagen mittels keramischer Farben werden Siebdrucköle eingesetzt, die ein Verlaufen der Farbpasten nach dem Druck verhindern und konturenscharfe Drucke ergeben. Dazu verwendet man Zusätze zu den bekannten Bindemitteln, die aus feinverteilten natürlichen oder künstlichen Wachsen und/oder aus feinverteilten, beim Brennen in das silikatische Gerüst des Flußmittels einbaufähigen, anorganischen silikatischen oder oxidischen Stoffen bestehen. Das Interferenzpigment gemäß Beispiel 1 wird mit der entsprechenden Menge Fritte und dem Druckmedium (in den Beispielen werden Siebdrucköl 221-ME und Screenprint Bulk 803035 MR - beides handelsübliche Produkte von Ferro - eingesetzt) für eine Reihe von Versuchen (siehe Tabelle 1) eingewogen und homogenisiert.

In den nachfolgenden Beispielen 1 bis 19 wird eine Fritte mit der nachfolgenden Zusammensetzung

| Fritte | CaO | $Na_2O$ | $K_2O$ | BaO | $Al_2O_3$ | $SiO_2$ | $B_2O_3$ |
|--------|-----|---------|--------|-----|-----------|---------|----------|
| Gew.% | 9,7 | 5,2 | 1,1 | 1,3 | 10,1 | 69,6 | 3,0 |

eingewogen und homogenisiert.

Die Einwaage der entsprechenden Rohstoffe zur Herstellung der Druckpaste, d.h. Interferenzpigment, Fritte und Drucköl zur Pastenherstellung ist der nachfolgenden Tabelle zu entnehmen:

Tabelle 1:

| Beispiel Nr. | InterferenzPigment (Beispiel 1) | Fritte | Drucköl | Anpast-Verhältnis | $W_{Pigm}$ im Feststoff | $W_{Pigm}$ in der Paste |
|--------------|--------------------------------|--------|---------|-------------------|-------------------------|-------------------------|
| 1 | 1,5 g | 3,5 g | 7,0 g | 10:14 | 30,00 | 12,50 |
| 2 | 1,0 g | 3,5 g | 7,0 g | 10:15,6 | 22,22 | 8,70 |
| 3 | 0,8 g | 3,5 g | 7,0 g | 10:16,3 | 18,60 | 7,08 |
| 4 | 0,6 g | 3,5 g | 7,0 g | 10:17,1 | 14,63 | 5,41 |
| 5 | 1,5 g | 3,5 g | 9,0 g | 10:18 | 30,00 | 10,71 |
| 6 | 1,0 g | 3,5 g | 9,0 g | 10:20 | 22,22 | 7,41 |
| 7 | 0,8 g | 3,5 g | 9,0 g | 10:20,9 | 18,60 | 6,02 |
| 8 | 0,6 g | 3,5 g | 9,0 g | 10:22 | 14,63 | 4,58 |
| 9 | 1,5 g | 2,0 g | 7,0 g | 10:20 | 42,86 | 14,29 |
| 10 | 1,5 g | 1,5 g | 7,0 g | 10:23,3 | 50,00 | 15,00 |
| 11 | 1,5 g | 1,0 g | 7,0 g | 10:28 | 60,00 | 15,79 |
| 12 | 1,5 g | 0,5 g | 7,0 g | 10:35 | 75,00 | 16,67 |
| 13 | 5,0 g | 0,0 g | 7,0 g | 10:14 | 100,00 | 41,67 |
| 14 | 2,0 g | 3,0 g | 7,0 g | 10:14 | 40,00 | 16,67 |
| 15 | 3,8 g | 1,2 g | 7,0 g | 10:14 | 76,00 | 31,67 |
| 16 | 4,3 g | 0,7 g | 7,0 g | 10:14 | 86,00 | 35,83 |
| 17 | 4,5 g | 0,5 g | 7,0 g | 10:14 | 90,00 | 37,50 |
| 18 | 4,5 g | 0,2 g | 7,0 g | 10:14 | 96,00 | 40,00 |
| 19 | 4,8 g | 0,1 g | 7,0 g | 10:14 | 98,00 | 40,83 |

Die nachfolgenden Schritte 2-4 sind unabhängig von der Zusammensetzung der Druckpaste.

2) Bedrucken der Fliesen

Die erhaltene Druckpaste kann über gängige Druckverfahren, Schlickerverfahren, Sprühapplikation oder Transferdruck auf Fliesen aufgebracht werden. In allen Fällen wird die bedruckte Fliese im Trockenschrank oder Abzug bei Temperaturen von 60 - 110 °C getrocknet, um das enthaltene Lösungsmittel im Drucköl abzudampfen. Bei den erfindungsgemäßen Beispielen wird die Druckpaste mittels Rakel und Drucksieb auf die Fliesen aufgebracht.

3) Brennen der bedruckten Fliesen

Die bedruckte und getrocknete Fliese wird nun im Brennofen mittels eines Temperaturprofiles gemäß Abbildung 6 gebrannt.

180 min :      Heizen auf 1100 °C,

(fortgesetzt)

| 3 min : | Halten bei 1100 °C, |
|---|---|
| 120 min : | schnelles Abkühlen auf 600 °C, |
| 300 min : | langsames Abkühlen auf Raumtemperatur. |

Die glasierten Fliesen der Beispiele 1 bis 19 zeichnen sich dadurch aus, dass die gewünschten optischen Effekte in der Hochtemperaturanwendung > 1100 °C stabil und reproduzierbar zugänglich sind.

## Patentansprüche

1. Interferenzpigmente basierend auf $SiO_2$-Plättchen, **dadurch gekennzeichnet, dass** sie mit Kristalliten des alpha-$Fe_2O_3$ beschichtet sind.

2. Interferenzpigmente nach Anspruch 1, **dadurch gekennzeichnet, dass** die $SiO_2$-Plättchen einen Durchmesser von 1-250 µm aufweisen.

3. Interferenzpigmente nach Anspruch 1 oder 2, **dadurch gekennzeichnet, dass** die $SiO_2$-Plättchen eine Dicke von 250-500 nm haben.

4. Interferenzpigmente nach einem oder mehreren der Ansprüche 1 bis 3, **dadurch gekennzeichnet, dass** die mittlere Kristallit-Größe des alpha-$Fe_2O_3 \geq 45$ nm beträgt.

5. Interferenzpigmente nach einem oder mehreren der Ansprüche 1 bis 4, **dadurch gekennzeichnet, dass** die mittlere Gesamtdicke der Interferenzpigmente bei 494 nm +/- 109 nm liegt.

6. Interferenzpigmente nach einem oder mehreren der Ansprüche 1 bis 5, **dadurch gekennzeichnet, dass** auf den Interferenzpigmenten zusätzlich eine organische oder anorganische Nachbeschichtung aufgebracht ist.

7. Verfahren zur Herstellung der Interferenzpigmente nach einem oder mehreren der Ansprüche 1 bis 6, **dadurch gekennzeichnet, dass** die Beschichtung der $SiO_2$-Plättchen mit den Kristalliten des alpha-$Fe_2O_3$ nasschemisch, im Wirbelbett und/oder durch CVD oder PVD-Verfahren erfolgt.

8. Verwendung der Interferenzpigmente nach einem oder mehreren der Ansprüche 1 bis 6 in Farben, Lacken, Industrie- und Automobillacken, Druckfarben, Sicherheitsdruckfarben, Papier, Kunststoffen, Folien, kosmetischen Formulierungen, Knopfpasten, Pigmentgemischen, Trockenpräparaten oder Pigmentpräparationen, keramischen Materialien, keramischen Farben, Glasuren, Engoben, Emaillen und Gläsern, als Absorber zur Lasermarkierung von Kunststoffen, zur Lebensmitteleinfärbung, zur Veredlung von Lebensmitteln, in Lebensmittel- und Arzneimittelüberzügen, für Sicherheitsmerkmale in Dokumenten und Ausweisen, zur Saatguteinfärbung, für RADAR-Anwendungen, zur Farbgebung von Solarzellen.

9. Formulierungen enthaltend das Interferenzpigment nach einem oder mehreren der Ansprüche 1 bis 6.

10. Formulierungen nach Anspruch 9, **dadurch gekennzeichnet, dass** sie neben dem Interferenzpigment mindestens einen Bestandteil ausgewählt aus der Gruppe Absorptionsmittel, Adstringenzien, antimikrobiellen Stoffe, Antioxidantien, Antiperspirantien, Antischaummittel, Antischuppenwirkstoffe, Antistatika, Aromastoffe, Bindemittel, biologischen Zusatzstoffe, Bleichmittel, Chelatbildner, Desodorierungsmittel, Drucköle, Emollentien, Emulgatoren, Emulsionsstabilisatoren, Farbstoffe, Feuchthaltemittel, Filmbildner, Fritten, Füllstoffe, Geruchsstoffe, Geschmacksstoffe, Insect Repellents, Konservierungsmittel, Korrosionsschutzmittel, kosmetischen Öle, Lösungsmittel, Oxidationsmittel, Parfüme, pflanzlichen Bestandteile, Proteine, Puffersubstanzen, Reduktionsmittel, Schleifmittel, Süßstoffe, Tenside, Treibgase, Trübungsmittel, UV-Filter, UV-Absorber, Vergällungsmittel, Aloe Vera, Avocadoöl, Coenzym Q10, grüner Tee Extrakt, organische Pigmente, anorganische Pigmente, Viskositätsregler, Vitamine, Enzyme, Spurenelemente, Kohlenhydrate enthalten.

Europäisches
Patentamt

European
Patent Office

Office européen
des brevets

**EUROPÄISCHER RECHERCHENBERICHT**

Nummer der Anmeldung

EP 25 19 1356

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X,D | EP 1 681 318 A2 (MERCK PATENT GMBH [DE]) 19. Juli 2006 (2006-07-19) * Ansprüche 1-10 * * Absätze [0001], [0006] - [0031] * * Beispiel 1 * ----- | 1-10 | INV. C09C1/00 A61K8/19 C04B35/00 C08K3/01 C08K3/013 C09D5/36 |
| X,D | WO 2007/057111 A2 (MERCK PATENT GMBH [DE]; PFAFF GERHARD [DE] ET AL.) 24. Mai 2007 (2007-05-24) * Seite 1, Zeilen 4-8 * * Seite 2, Zeile 23 - Seite 19 * * Beispiel 1 * ----- | 1,3-10 | |

RECHERCHIERTE
SACHGEBIETE (IPC)

C09C
C09G
C08K
A61Q
A61K
C04B
C09D

Der vorliegende Recherchenbericht wurde für alle Patentansprüche erstellt

| Recherchenort | Abschlußdatum der Recherche | Prüfer |
|---|---|---|
| Den Haag | 9. Januar 2026 | Marino, Emanuela |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument
.......................................................................
& : Mitglied der gleichen Patentfamilie, übereinstimmendes
    Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 25 19 1356

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

09-01-2026

| Im Recherchenbericht angeführtes Patentdokument | Datum der Veröffentlichung | Mitglied(er) der Patentfamilie | Datum der Veröffentlichung |
|---|---|---|---|
| EP 1681318 A2 | 19-07-2006 | CN 1814673 A | 09-08-2006 |
| | | DE 102005002124 A1 | 27-07-2006 |
| | | EP 1681318 A2 | 19-07-2006 |
| | | ES 2428502 T3 | 08-11-2013 |
| | | JP 2006199960 A | 03-08-2006 |
| | | JP 2013100524 A | 23-05-2013 |
| | | KR 20060083892 A | 21-07-2006 |
| | | KR 20130081686 A | 17-07-2013 |
| | | TW I405822 B | 21-08-2013 |
| | | US 2006156949 A1 | 20-07-2006 |
| WO 2007057111 A2 | 24-05-2007 | CN 101309980 A | 19-11-2008 |
| | | DE 102005055576 A1 | 24-05-2007 |
| | | EP 1948737 A2 | 30-07-2008 |
| | | JP 2009516035 A | 16-04-2009 |
| | | JP 2015045002 A | 12-03-2015 |
| | | JP 2017088885 A | 25-05-2017 |
| | | JP 2019026854 A | 21-02-2019 |
| | | JP 2019026855 A | 21-02-2019 |
| | | TW I409304 B | 21-09-2013 |
| | | US 2009220557 A1 | 03-09-2009 |
| | | US 2011300200 A1 | 08-12-2011 |
| | | WO 2007057111 A2 | 24-05-2007 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 9308237 A
- WO 2007057111 A2
- EP 1681318 A2
- EP 0632109 A
- US 5759255 A
- DE 4317019
- DE 3929423
- DE 3235017
- EP 0492223 A
- EP 0342533 A
- EP 0268918 A
- EP 0141174 A
- EP 0764191 A
- WO 9813426 A
- EP 0465805 A
- EP 0090259 A
- EP 0634459 A
- WO 9957204 A
- WO 9632446 A
- US 5571851 A
- WO 0192425 A
- EP 0671161 A
- DE 4308282 A

**In der Beschreibung aufgeführte Nicht-Patentliteratur**

- **J.J. PONJEÉ**. *Philips Technical Review*, vol. 44 (3), 81
- **P.H. HARDING** ; **J.C. BERG**. *J. Adhesion Sci. Technol.*, vol. 11 (4), 471-493
- *CHEMICAL ABSTRACTS*, 13822-56-5
- *CHEMICAL ABSTRACTS*, 2530-83-8
- *CHEMICAL ABSTRACTS*, 3069-19-0